# EUROPÄISCHE PATENTANMELDUNG

(11) **EP 4 851 624 A1**
(43) Veröffentlichungstag der Anmeldung: **09.12.2026**
(21) Anmeldenummer: 26178578.6
(22) Anmeldetag: 13.05.2026
(51) Int. Cl.: G01N 33/00, G01N 27/403

(54) **ELEKTROCHEMISCHER GASSENSOR UND GEHÄUSE**

(30) Priorität: 03.06.2025 DE 102025121628
(71) Anmelder: Testo SE & Co. KGaA, 79822 Titisee-Neustadt (DE)
(72) Erfinder: Stallmann, Siegfried, 79848 Bonndorf (DE); Schirmaier, Stefan, 79102 Freiburg (DE)
(74) Vertreter: Maucher Jenkins Patent- und Rechtsanwälte Partnerschaft mbB

(57) **Zusammenfassung**

Gehäuse (2) für einen elektrochemischen Gassensor (1) mit einem Innenraum (3), wobei das Gehäuse (2) eine Kapillare(8) aufweist, die zum Abführen von im Innenraum (3) entstehendem Gas aus dem Gehäuse (2) dient,
dadurch gekennzeichnet, dass die Kapillare(8) zumindest teilweise durch wenigstens zwei Bauteile gebildet ist,
wobei ein erstes Bauteil eine Nut (11) aufweist, die in zumindest einer Fläche (12) des ersten Bauteils angeordnet ist und ein zweites Bauteil (13), durch das in Gebrauchsstellung die Nut (11) zumindest teilweise zugedeckt ist.

## Beschreibung

Die Erfindung umfasst ein Gehäuse für einen elektrochemischen Gassensor und einen elektrochemischen Gassensor.

Ein elektrochemischer Gassensor weist in der Regel ein Gehäuse mit einem Innenraum auf. In dem Innenraum sind mehrere Elektroden, weitere Elemente wie Separatoren und/oder Membranen und ein Elektrolyt, beispielsweise Schwefelsäure angeordnet. Im Betrieb des Sensors kann Wasserstoff gebildet werden, wodurch ein Überdruck im Gehäuse entstehen kann, durch den beispielsweise das Gehäuse platzen oder undicht werden kann.

Um diesen Überdruck zu verhindern, besitzt ein elektrochemischer Sensor oder das Gehäuse eine Kapillare, also eine Öffnung nach außen, über die eventuell entstehendes Gas entweichen kann.

Unter bestimmten Betriebsbedingungen kann es jedoch dazu kommen, dass der Elektrolyt, also beispielsweise Schwefelsäure, aus der Kapillare austreten kann. Aufgabe der Erfindung ist es, einen solchen Schwefelsäureaustritt zu verhindern.

Diese Aufgabe wird erfindungsgemäß durch ein Gehäuse und einen elektrochemischen Sensor gemäß der unabhängigen Ansprüche gelöst.

Das erfindungsgemäße Gehäuse ist demnach dadurch gekennzeichnet, dass die Kapillare zumindest teilweise durch wenigstens zwei Bauteile gebildet ist, wobei ein erstes Bauteil eine Nut aufweist, die in zumindest einer Fläche des ersten Bauteils angeordnet ist und ein zweites Bauteil, durch das in Gebrauchsstellung die Nut zumindest teilweise zugedeckt ist. Der Vorteil dabei liegt darin, dass eine Nut einfacher herstellbar ist als beispielsweise eine Bohrung oder eine sonstige Öffnung im Gehäuse.

Besonders vorteilhaft kann es sein, wenn die Kapillare ein bestimmtes Aspektverhältnis aufweist. Durch die zweiteilige Bauweise der Kapillare ist es besonders einfach und kostengünstig, ein solches Aspektverhältnis zu erzielen.

Von Vorteil ist dabei, dass ein Elektrolyt, etwa Schwefelsäure, dadurch gehindert werden kann aus dem Gehäuse auszutreten.

Besonders vorteilhaft ist es, wenn die Kapillare ein Aspektverhältnis von Durchmesser zu Länge von höchstens 1/15 besitzt, vorzugsweise liegt dieses Aspektverhältnis zwischen 0,01 und 0,04. Durch dieses Aspektverhältnis der Nut kann eine Länge erzielt werden, durch die ein Austritt von Elektrolyt vollständig verhindert werden kann.

In einer Ausführung besitzt die Nut einen halbkreisförmigen Querschnitt. Die nicht planen Flächen der Nut können beispielsweise im Spritzguss einfacher entformt werden, wodurch etwa ein kleineres Aspektverhältnis erzielbar ist. Auf diese Weise ist eine einfache Herstellung möglich.

In einer Ausführung ist die Nut als stetige Kurve, insbesondere spiralartig, ausgebildet. Auf diese Weise kann auf einer gegebenen Grundfläche eine größere Gesamtlänge der Nut erzielt werden, wodurch wiederum ein günstiges Aspektverhältnis erzielbar ist.

In einer Ausführung verläuft die Nut in einer Ebene, die zumindest teilweise parallel zu einer Querschnittsebene des Innenraums liegt. Auf diese Weise ist eine besonders einfache Herstellung möglich.

Hierbei kann der Innenraum des Gehäuses einen kreisförmigen Querschnitt aufweisen. Der Vorteil besteht darin, dass kreisförmige Elektroden verwendbar sind, und ein möglichst kompaktes Gehäuse erzielbar ist.

In einer Ausführung ist das erste Bauteil ein Gehäuseteil. Auf diese Weise kann die Nut ohne zusätzlichen Herstellungsprozess direkt bei der Herstellung des Gehäuses erstellt werden.

In einer vorteilhaften Ausführung bildet das erste Bauteil den Innenraum und die Nut ist in einer Fläche angeordnet, die außerhalb des Innenraums liegt. Vorzugsweise liegt die Fläche parallel zu einer Fläche, die den Innenraum begrenzt. Insbesondere begrenzen die beiden Flächen eine Wand des Innenraums, beispielsweise den Boden des Innenraums.

Besonders vorteilhaft ist es, wenn das erste Bauteil als ein Spritzgussteil hergestellt ist. Auf diese Weise ist eine besonders kostengünstige und einfache Herstellung ermöglicht.

In einer Ausführung weist das zweite Bauteil eine Gegenfläche auf, die in Gebrauchsstellung plan auf der wenigstens einen Fläche des ersten Bauteils aufliegt. Auf diese Weise wird die Nut durch die Gegenfläche des zweiten Bauteils abgedeckt und so eine Kapillare gebildet.

In einer alternativen Ausführung ist in dem zweiten Bauteil, insbesondere in der Gegenfläche, eine korrespondierende Nut angeordnet, so dass nach dem Zusammenfügen der beiden Bauteile durch die beiden Nuten die Kapillare gebildet ist.

In einer Ausführung ist das zweite Bauteil als eine Abdeckfolie ausgebildet, die auf die Fläche des ersten Bauteils aufgesetzt ist. Der Vorteil besteht darin, dass eine solche Folie kostengünstig ist und sehr einfach montierbar ist. Darüber hinaus erfordert die Folie nur eine geringe Bauhöhe.

Insbesondere wenn die Fläche mit der Nut des ersten Bauteils den Innenraum begrenzt, ist eine solche Abdeckfolie vorteilhaft, da dadurch nur eine geringe Bauhöhe des Innenraums benötigt wird. Somit kann eine geringe Bauhöhe des Sensors erzielt werden.

Die Abdeckfolie kann vorteilhaft als eine selbstklebende Folie ausgebildet sein.

In einer Ausführung ist die Abdeckfolie kreisförmig ausgebildet und weist eine Abflachung auf, wobei durch die Abflachung eine Öffnung der Nut nach Außen erzielt wird.

In einer Ausführung ist ein erstes Ende der Nut durch eine Kapillaröffnung mit dem Innenraum verbunden. Diese Kapillaröffnung kann etwa eine Bohrung oder eine Aussparung im Gehäuse sein.

In jedem Fall ist es vorteilhaft, wenn das erste Bauteil und das zweite Bauteil separate Bauteile sind. Die beiden Bauteile können dabei auch aus verschiedenen Materialien bestehen.

Die Erfindung umfasst auch einen elektrochemischen Gassensor mit einem Gehäuse, das einen Innenraum aufweist, wobei der Gassensor eine Kapillare aufweist, die zum Abführen von im Innenraum entstehendem Gas aus dem Gehäuse dient.

Erfindungsgemäß ist die Kapillare zumindest teilweise durch wenigstens zwei Bauteile gebildet, wobei ein erstes Bauteil eine Nut aufweist, die in zumindest einer Fläche des ersten Bauteils angeordnet ist und ein zweites Bauteil, durch das in Gebrauchsstellung die Nut zumindest teilweise zugedeckt ist.

In einer solchen Ausführung liegt die durch die Nut gebildete Kapillare innerhalb des Innenraums.

Dabei ist es auch möglich, dass beispielsweise die Nut nicht Teil des Gehäuses ist, sondern beispielsweise in einer Elektrode oder einer Trägermembran einer Elektrode angeordnet ist. Diese kann beim Einlegen in den Innenraum des Gehäuses mit einer Gegenfläche des Gehäuses die Kapillare bilden.

In einer Ausführung ist das erste Bauteil ein Gehäusebauteil und das zweite Bauteil ist gebildet durch eine Elektrode, einen Träger für eine Elektrode oder einen Separator. Auf diese Weise kann beispielsweise auf eine oben beschriebene Abdeckfolie verzichtet werden, wodurch der Aufbau des Sensors zusätzlich vereinfacht ist.

Alternativ kann auch die Kapillare außerhalb des Innenraums dadurch gebildet sein, dass das erste Bauteil, in dem die Nut angeordnet ist, kein Gehäusebauteil ist. In diesem Fall kann die Nut beispielsweise in einem separaten Anlegeteil angeordnet sein, das auf eine Gegenfläche an dem Gehäuse angelegt wird.

Insbesondere vorteilhaft ist es, wenn das Gehäuse des elektrochemischen Sensors gemäß der oben beschriebenen Erfindung ausgebildet ist.

Die Erfindung ist nachfolgend anhand von Ausführungsbeispielen mit Bezug auf die beiliegenden Zeichnungen näher erläutert.

Es zeigt:
- Fig. 1: eine Schnittansicht eines elektrochemischen Sensors,
- Fig. 2: eine weitere Schnittansicht des elektrochemischen Sensors der Fig. 1,
- Fig. 3: eine Draufsicht einer Fläche des elektrochemischen Sensors der Fig. 1 mit einer Nut,
- Fig. 4: eine Draufsicht eines elektrochemischen Sensors der Fig. 1 mit einer Abdeckfolie, und
- Fig. 5: die Draufsicht der Fig. 4 verdreht angeordneter Abdeckfolie.

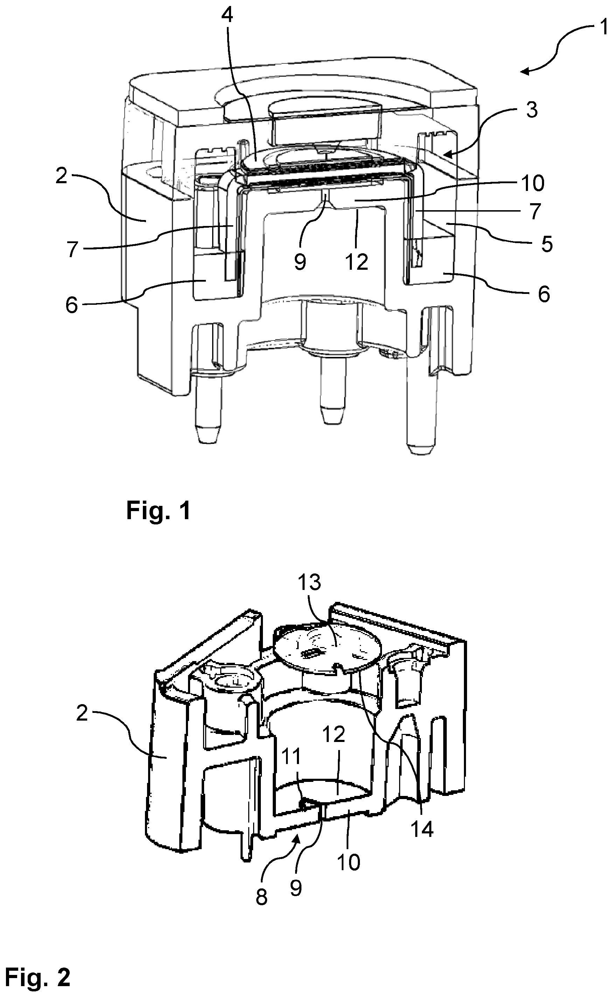

Die Figuren 1 und 2 zeigen verschiedene Schnittansichten eines elektrochemischen Sensors 1 mit einem Gehäuse 2. Das Gehäuse 2 begrenzt einen Innenraum 3, in dem mehrere Elektroden 4 angeordnet sind. Das Gehäuse 2 weist weiter ein Reservoir 5 auf, in dem Schwefelsäure 6 vorgehalten ist. Zwischen den Elektroden 4 ist ein Vlies 7 angeordnet, das in die Schwefelsäure 6 eintaucht. Der Aufbau eines elektrochemischen Sensors ist im Stand der Technik bekannt und für die Erfindung nicht wesentlich, weshalb auf eine genauere Beschreibung verzichtet wird.

Das Gehäuse 2 weist weiter eine Kapillare8 auf, die zur Entlüftung des Innenraums 3 dient. Die Kapillare 8 weist eine Kapillaröffnung 9 auf, die in dem Boden 10 des Innenraums 3 angeordnet ist. Die Kapillaröffnung 9 verbindet den Innenraum 3 mit einer Nut 11. Die Nut 11 ist auf der anderen Seite des Bodens 10 auf einer Fläche 12, die außerhalb des Innenraums 3 liegt, angeordnet.

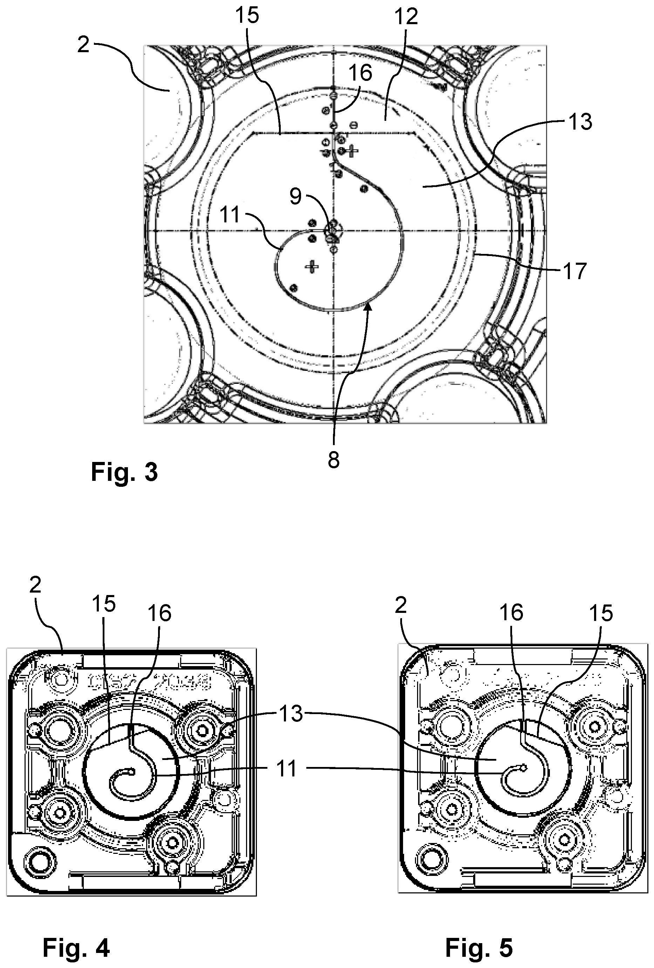

Die Fig. 3 zeigt ausschnittsweise die außen liegende Fläche 12 des Bodens 10. Die Kapillaröffnung 9 ist etwa in der Mitte der Fläche 12 angeordnet. Ausgehend von der Kapillaröffnung 9 verläuft eine Nut 11 in etwa spiralartig zu einem Rand der Fläche 12.

Eine Abdeckfolie 13 liegt mit einer Gegenfläche 14 auf dieser Fläche 12 auf, so dass die Nut 11 abgedeckt ist. Die Abdeckfolie 13 weist eine Abflachung 15 auf, durch die die Nut 11 an ihrem freien Ende 16 offen ist. Dadurch ist die Nut 11 zur Außenumgebung des Sensors 1 geöffnet. Die Abdeckfolie 13 kann dabei elastisch oder starr ausgebildet sein. Vorzugsweise ist die Abdeckfolie 13 als selbstklebende Folie ausgebildet.

Die Nut 11 weist ein vorteilhaftes Aspektverhältnis aus Durchmesser zu Länge auf, wodurch die Nut 11 zusammen mit der Abdeckfolie 13 die Kapillare bildet. Diese Kapillare verhindert, dass Lösungsmittel, beispielsweise Alkohol, in Gasform, in den Sensor eindringen und diesen zur Wasserstoffproduktion anregen kann.

Die Nut 11 ist sehr einfach in einem Spritzgussprozess herstellbar. Vorzugsweise besitzt die Nut 11 einen halbkreisförmigen Querschnitt. Dadurch besitzt die Nut 11 keine Wandung, die parallel zur Entformungsrichtung eines Spritzgusswerkzeugs liegt, wodurch die Herstellung zusätzlich vereinfacht sein kann.

Im gezeigten Beispiel liegt die Nut 11 innerhalb einer Fläche 12, die parallel zu einer Querschnittsfläche des Innenraums 3 liegt. Insbesondere liegt die Fläche 12 auch senkrecht zu einer Entformungsrichtung eines Spritzgusswerkzeugs. Die Länge der Nut und somit das Aspektverhältnis ist auf diese Weise beliebig festlegbar, da es für die Herstellung unerheblich ist.

Im Beispiel besitzt die Nut 11, wie bereits erwähnt, einen halbreisförmigen Querschnitt mit einem Radius von 0,15 mm. Die Nut ist somit 0,3 mm breit und 0,15 mm tief. Die abgedeckte Länge der Nut 11 beträgt 11 mm, wobei dies als minimale Länge angesehen werden kann, um einen wirksamen Schutz vor einem Austreten von Schwefelsäure zu gewährleisten.

Die Fig. 4 und 5 zeigen exemplarisch eine Lagetoleranz der Abdeckfolie 13 mit Abflachung 15. Das offene Ende 16 der Nut 11 liegt zweckmäßigerweise radial zu einem Innenkreis 17 der Fläche 12. Durch die Abflachung 15 ist eine Verdrehung der Abdeckfolie 13 im Bereich von ±20° gegenüber der Mittellage tolerierbar. Somit ist die Herstellung vereinfacht, da beim Anbringen der Abdeckfolie 13 nur eine geringe Genauigkeit erforderlich ist.

In dem gezeigten Beispiel ist die Nut in dem Gehäuse 2 des elektrochemischen Sensors 1 als erstem Bauteil angeordnet. Es wäre jedoch genauso möglich, die Nut 11 in der Abdeckfolie 13 oder einem zusätzlichen Bauteil anzuordnen, das anstelle der Abdeckfolie auf die Fläche 12 des Gehäuses aufsetzbar ist, um die Kapillare zu bilden.

In einer alternativen, nicht gezeigten Ausführung könnte die Nut auch im Innenraum, etwa der Innenfläche des Bodens 10 angeordnet sein. Dabei könnte eine der Elektroden 4 als Abdeckung dienen.

Es wäre auch möglich, dass die Nut über zwei Flächen eines Bauteils verläuft, etwa teilweise quer und teilweise parallel zu einer Entformungsrichtung eines Spritzgusswerkzeugs.

### Bezugszeichenliste

- 1: elektrochemischer Sensor
- 2: Gehäuse
- 3: Innenraum
- 4: Elektroden
- 5: Reservoir
- 6: Schwefelsäure
- 7: Vlies
- 8: Kapillare
- 9: Kapillaröffnung
- 10: Boden
- 11: Nut
- 12: Fläche
- 13: Abdeckfolie
- 14: Gegenfläche
- 15: Abflachung
- 16: Freies/offenes/zweites Ende der Nut
- 17: Innenkreis

## Patentansprüche

1. Gehäuse (2) für einen elektrochemischen Gassensor (1) mit einem Innenraum (3), wobei das Gehäuse (2) eine Kapillare(8) aufweist, die zum Abführen von im Innenraum (3) entstehendem Gas aus dem Gehäuse (2) dient,
**dadurch gekennzeichnet, dass** die Kapillare(8) zumindest teilweise durch wenigstens zwei Bauteile gebildet ist, wobei ein erstes Bauteil eine Nut (11) aufweist, die in zumindest einer Fläche (12) des ersten Bauteils angeordnet ist und
ein zweites Bauteil (13), durch das in Gebrauchsstellung die Nut (11) zumindest teilweise zugedeckt ist.

2. Gehäuse (2) nach Anspruch 1, **dadurch gekennzeichnet, dass** die Kapillare durch die Nut (11) gebildet ist, insbesondere wobei die Kapillare (18) ein Aspektverhältnis von Durchmesser zu Länge von höchstens 1/15 besitzt, vorzugsweise wobei dieses Verhältnis zwischen 0,01 und 0,04 liegt.

3. Gehäuse (2) nach einem der vorangehenden Ansprüche, **dadurch gekennzeichnet, dass** die Nut (11) einen halbkreisförmigen Querschnitt besitzt und/oder dass die Nut (11) als stetige Kurve, insbesondere spiralartig, ausgebildet ist.

4. Gehäuse (2) nach einem der vorangehenden Ansprüche, **dadurch gekennzeichnet, dass** die Nut (11) zumindest teilweise parallel zu einer Querschnittsebene des Innenraums (3) verläuft, insbesondere wobei der Innenraum (3) einen kreisförmigen Querschnitt aufweist und/oder dass die Nut (11) zumindest teilweise außerhalb des Innenraums (3) angeordnet ist.

5. Gehäuse (2) nach einem der vorangehenden Ansprüche, **dadurch gekennzeichnet, dass** das erste Bauteil ein Gehäuseteil ist, insbesondere wobei das erste Bauteil den Innenraum bildet und die Nut (11) in einer Fläche (12), die außerhalb des Innenraums (3) liegt, angeordnet ist, und insbesondere wobei das erste Bauteil (2) als ein Spritzgussteil hergestellt ist.

6. Gehäuse (2) nach einem der vorangehenden Ansprüche, **dadurch gekennzeichnet, dass** das zweite Bauteil eine Gegenfläche aufweist, die in Gebrauchsstellung plan auf der wenigstens einen Fläche des ersten Bauteils aufliegt, und/oder wobei in dem zweiten Bauteil eine korrespondierende Nut angeordnet ist, so dass nach dem Zusammenfügen der beiden Bauteile durch die beiden Nuten die Kapillare gebildet ist.

7. Gehäuse (2) nach einem der vorangehenden Ansprüche, **dadurch gekennzeichnet, dass** das zweite Bauteil als eine Abdeckfolie ausgebildet ist, die auf die Fläche des ersten Bauteils aufgesetzt ist, insbesondere wobei die Abdeckfolie (13) kreisförmig ausgebildet ist und eine Abflachung (15) aufweist, wobei durch die Abflachung (15) eine Öffnung der Nut zur Umgebung hergestellt ist.

8. Gehäuse (2) nach einem der vorangehenden Ansprüche, **dadurch gekennzeichnet, dass** ein erstes Ende der Nut (11) durch eine Kapillaröffnung (9) mit dem Innenraum (3) des Gehäuses (2) verbunden ist und/oder dass ein zweites Ende (16) der Nut nach Außen geöffnet ist.

9. Elektrochemischer Gassensor (1) mit einem Gehäuse (2), das einen Innenraum (3) aufweist, wobei der Gassensor (1) eine Kapillare(8) aufweist, die zum Abführen von im Innenraum (1) entstehendem Gas aus dem Gehäuse (2) dient,
**dadurch gekennzeichnet, dass** die Kapillare (8) durch wenigstens zwei Bauteile gebildet ist,
wobei ein erstes Bauteil (2) eine Nut (11) aufweist, die in zumindest einer Fläche (12) des ersten Bauteils (2) angeordnet ist und
ein zweites Bauteil (13), durch das in Gebrauchsstellung die Nut (11) zumindest teilweise zugedeckt ist.

10. Elektrochemischer Gassensor nach Anspruch 9, **dadurch gekennzeichnet, dass** das erste Bauteil (2) ein Gehäusebauteil ist und das zweite Bauteil durch eine Elektrode, einen Träger für eine Elektrode oder einen Separator gebildet ist und/oder dass das Gehäuse (2) nach einem der Ansprüche 2 bis 8 ausgebildet ist.
